# EUROPÄISCHE PATENTSCHRIFT

(11) **EP 1 323 439 B1**
(45) Veröffentlichungstag und Bekanntmachung des Hinweises auf die Patenterteilung: **23.01.2008**
(21) Anmeldenummer: 03005856.4
(22) Anmeldetag: 11.03.1993
(51) Int. Cl.: A61M 1/36

(54) **Anordnung zum On-Line Spülen und Befüllen oder Freispülen eines extrakorporalen Blutkreislaufs von Dialysemaschinen**
Device for on-line cleaning and filling or emptying of an extra corporeal blood circuit of a dialysis apparatus
Dispositif de nettoyage et remplissage ou vidange en ligne d'un circuit extracorporel sanguin d'une machine de dialyse

(30) Priorität: 13.03.1992 DE 4208274
(43) Veröffentlichungstag der Anmeldung: 02.07.2003
(62) Teilanmeldung aus: 97118498.1
(73) Patentinhaber: Fresenius Medical Care, 61352 Bad Homburg (DE)
(72) Erfinder: Eigendorf, Hans-Günther, Dr., 15526 Bad Saarow-Pieskow (DE)
(74) Vertreter: Flosdorff, Jürgen

(56) Entgegenhaltungen:
- FR-A- 2 175 945
- US-A- 4 784 771
- US-A- 4 840 769

## Beschreibung

Die Erfindung betrifft eine Anordnung zum Freispülen eines extrakorporalen Blutkreislaufs von Dialysemaschinen, insbesondere von Blutschlauchleitungen und Dialysatoren, nach Beendigung einer Dialysebehandlung, wobei von der Dialysemaschine erzeugte bzw. aufbereitete Dialysierflüssigkeit durch den bzw. in den extrakorporalen Blutkreislauf gefördert wird.

Als arterielle und venöse Blutschlauchleitung eines extrakorporalen Blutkreislaufs werden heutzutage üblicherweise steril verpackte Einmalartikel verwendet, die vor einer Behandlung gespült, entlüftet und befüllt werden müssen. Dies geschieht bisher mittels physiologischer Kochsalzlösungen, die mittels der maschineneigenen Blutpumpe durch das Schlauchsystem und den Dialysator gefördert werden, um den extrakorporalen Blutkreis- lauf von Verunreinigungen freizuspülen und unter vollständiger Entlüftung zu befüllen.

Die Verwendung der Kochsalzspülflüssigkeit hat den Nachteil, daß sie mit erheblichen Kosten verbunden ist.

Die FR 2 175 945 A offenbart ein Freispülverfahren eines extrakoporalen Blutkreislaufs nach Beendigung einer Dialysebehandlung, bei dem das Ende einer Blutschlauchleitung mit einer Salzlösung verbunden wird, so daß bei laufender Pumpe das Blut aus dem Dialysator entfernt wird. Anschließend werden die Anschlußenden der Blutschlauchleitungen an Anschlüssen von Verbindungsleitungen angebracht, wobei eine Verbindungsleitung Dialysat aus einem Dialysatbehälter zuführt und die andere Verbindungsleitung das Dialysat in einen Aufnahmebehälter abführt. Bei weiterlaufender Pumpe spült das Dialysat den Blutkreislauf.

Die US 4,784,771 offenbart eine Vorrichtung und ein Verfahren zum Reinigen und/oder Trennen von Fluiden, wobei das Fluid von einer Beschickungsseite von einer Umkehrosmoseeinheit zu der Produktseite der Einheit und zu einem Verwender während eines Reinigungszyklus verläuft und restliches Fluid durch einen Bypaß zu der Beschickungsseite der Umkehrosmoseeinheit während eines Behandlungszyklus rezirkuliert wird, um geeignete Behandlungsfluide zum Desinfizieren des Systems einzuführen.

Der vorliegenden Erfindung liegt die Aufgabe zugrunde, eine Anordnung anzugeben, mit der das Freispülen eines extrakorporalen Blutkreislaufs von Dialysemaschinen vereinfacht ist.

Diese Aufgabe wird erfindungsgemäß durch die Merkmale des Anspruchs 1 gelöst.

Die Erfindung sieht vor, daß mit einem automatisch ablaufenden Programm im Rahmen des Steuerprogramms der Dialysemaschine Dialysierflüssigkeit durch die Membran des Dialysators hindurch mit der maschineneigenen Blutpumpe sowie Mitteln zur Erzeugung eines erhöhten Drucks in der Dialysatkammer gegenüber der Blutkammer des Dialysators in den extrakorporalen Blutkreislauf förderbar ist.

Somit wird die von der Dialysemaschine erzeugte bzw. aufbereitete Dialysierflüssigkeit zum Zwecke des Freispülens durch die Membran des Dialysators hindurch in den extrakorporalen Blutkreislauf gefördert. Hierdurch entfällt das bisherige Erfordernis, eine in einem Beutel abgefüllte Kochsalzlösung zu diesem Zweck bereit zu stellen, der zudem von einer Bedienungsperson an die arterielle Blutschlauchleitung anzuschließen war. Es entfällt damit ferner das Erfordernis, zu dem genannten Zweck Kochsalzlösungen in größeren Mengen bereit zu halten und die geleerten Beutel zu entsorgen.

Die Dialysierflüssigkeit wird beim Durchtritt durch die Membran filtiert. Hiermit ist die Keimfreiheit der Freispülflüssigkeit zuverlässig gewährleistet.

Wenn die Blutpumpe in Betrieb gesetzt wird, wird Dialysierflüssigkeit über die semipermeable Membran des Dialysators in die Blutschlauchleitungen gezogen. Dabei gelangt nur sterile und keimfreie Spülflüssigkeit in den extrakorporalen Blutkreislauf.

Die Erfindung sieht vor, daß nach dem Konnektieren der arteriellen und venösen Blutschlauchleitung am Dialysator die Patientenanschlüsse dieser beiden Leitungen miteinander verbunden werden, um eine durchgehende Flüssigkeitsleitung zu bilden, daß der Strömungsweg zwischen dem venösen Blutanschluß des Dialysators und der zur Entlüftung vorzugsweise vorgesehenen venösen Tropfkammer gesperrt wird, daß die Blutpumpe entgegen ihrer normalen Förderrichtung in Betrieb gesetzt wird, und daß die aus dem Blutschlauchsystem verdrängte Luft und überschüssige Flüssigkeit durch eine mit dem oberen Teil der venösen Tropfkammer verbundene Leitung abgeleitet wird. Dabei kann vorgesehen sein, daß die verdrängte Luft und die überschüssige Flüssigkeit in das Dialysierflüssigkeitsystem des Dialysegerätes abgeführt werden.

Dies kann dadurch weiter ausgestaltet werden, daß intermittierend die normalen Strömungsverhältnisse des extrakorporalen Blutkreislaufs hergestellt werden, d.h. öffnen des Strömungsweges zwischen dem venösen Blutanschluß des Dialysators und der venösen Tropfkammer und Inbetriebsetzen der Blutpumpe in ihrer normalen Förderrichtung, wobei die mit dem oberen Teil der venösen Tropfkammer verbundene Leitung verschlossen ist.

Das Umschalten der Ventile und der Förderrichtung der Blutpumpe kann über ein automatisch ablaufendes Programm im Rahmen des Steuerungsprogramms des Dialysegerätes bewirkt werden.

Bei einer Variante werden der arterielle und der venöse Patientenanschluß der Blutschlauchleitungen zur Schaffung einer durchgehenden Flüssigkeitsleitung miteinander verbunden, und eine an den oberen Teil der venösen Tropfkammer angeschlossene Leitung wird mit einer Saugvorrichtung versehen bzw. verbunden. Hierbei kann als Saugvorrichtung das Entgasungssystem des Dialysegerätes verwendet werden, wobei alternativ eine Pumpe angeordnet sein kann, deren abfördernde Leitung mit dem Dialysierflüssigkeitssystem verbunden sein kann.

Es ist vorgesehen, daß in der Dialysatkammer des Dialysators in vorgegebenen Zeitabständen ein erhöhter Druck erzeugt wird, der den Druck in der Blutkammer des Dialysators übersteigt und eine Filtration von Dialysierflüssigkeit in das Blut bewirkt. Dieser nach dem Anschließen eines Patienten und dem Beginn der eigentlichen Behandlung erfolgende Vorgang dient dazu, die Dialysemembran entgegen der normalen Filtrationsrichtung periodisch freizuspülen, d. h. Ablagerungen, die sich auf der Blutseite der Membran angesammelt haben, zu entfernen.

Vorzugsweise wird hierbei ein volumenstarr wirkendes Dialysierflüssigkeitssystem verwendet, wodurch die Möglichkeit geschaffen ist, daß eine periodische Druckerhöhung und Druckabsenkung mit der Pumpe bewirkt wird, die abwechselnd zusätzliche Flüssigkeit in das Dialysierflüssigkeitssystem einführt bzw. dem Dialysierflüssigkeitssystem Flüssigkeit entzieht, was in näheren Einzelheiten weiter unten noch beschrieben wird.

Erfindungsgemäß kann diese Vorgehensweise in Verbindung mit einer Hämodiafiltration angewendet werden.

Nach einem zusätzlichen Vorschlag der Erfindung ist vorgesehen, daß ein Sterilfilter stromaufwärts des Dialysators in dem Dialysierflüssigkeitskreislauf angeordnet wird, um die Sicherheit in Bezug auf die erforderliche Keimfreiheit zusätzlich zu erhöhen.

Nach einem weiteren Gesichtspunkt der Erfindung wird vorgeschlagen, daß die als Spülflüssigkeit verwendete Dialysierflüssigkeit nach Beendigung einer Dialysebehandlung zur Freispülung des extrakorporalen Blutkreislaufes verwendet wird, wozu es zweckmäßig ist, diese Flüssigkeit beim Spülvorgang in einen Beutel abzuführen. Das Freispülen ist notwendig, um nach der Dialysebehandlung das Blut vollständig zum Patienten zurückzuführen, was bisher ebenfalls mit Hilfe einer Kochsalzlösung geschieht.

Nach einem weiteren Vorschlag der Erfindung verbindet eine Leitung den oberen Teil der venösen Tropfkammer und das Dialysierflüssigkeitssystem, wobei diese Leitung mit einer Pumpe und zwischen dieser Pumpe und dem oberen Teil der venösen Tropfkammer mit einem Puffergefäß sowie einem Ventil zum öffnen/Schlie-Ben des Leitungsabschnitts zwischen dem Puffergefäß und der venösen Tropfkammer versehen ist.

Ein weiterer erfindungsgemäßer Vorschlag geht dahin, die extrakorporalen Komponenten so zu reinigen, daß sie wiederverwendbar sind. Hierzu kann die Anordnung so getroffen werden, wie sie weiter oben im Zusammenhang mit der ersten Ausführungsform beschrieben ist. Bei einem dialysemaschinenspezifischen Reinigungsprogramm wird durch die Förderung der Blutpumpe Reinigungsflüssigkeit über die semipermeable Membran des Dialysators in den extrakorporalen Kreislauf gezogen, wodurch dieser entsprechend gereinigt wird.

Selbstverständlich liegt es im Rahmen der Erfindung, die extrakorporalen Komponeten weiterhin als Einmalartikel einzusetzen.

Weitere Merkmale, Vorteile und Einzelheiten der Erfindung ergeben sich aus der nachfolgenden Beschreibung einiger bevorzugter Ausführungsformen sowie anhand der Zeichnung. Dabei zeigen auf rein schematische Weise:
- Fig. 1: eine herkömmliche Anordnung;
- Fig. 2: eine erste Ausführungsform der erfindungsgemäßen Anordnung;
- Fig. 3: eine zweite Ausführungsform der erfindungsgemäßen Anordnung;
- Fig. 4: eine dritte Ausführungsform der erfindungsgemäßen Anordnung.

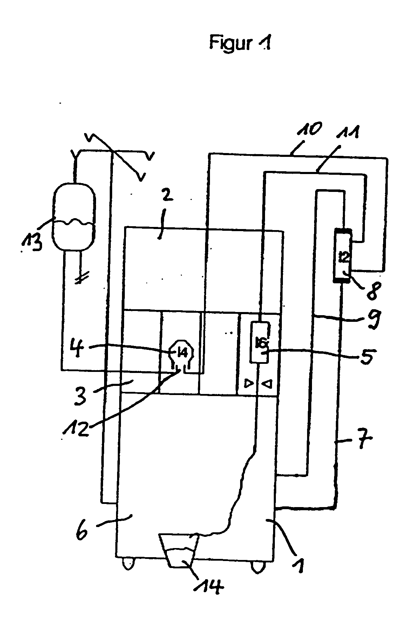

Es wird zunächst auf Fig. 1 Bezug genommen, um das bisher übliche Verfahren zum Spülen, Entlüften und Befüllen eines extrakorporalen Blutkreislaufs zu erläutern. Die Fig. zeigt eine eigentliche Dialysemaschine 1 mit der oberen Überwachungsebene 2, der mittleren Ebene 3, die eine Blutpumpe 4 und einen Luftdetektor 5 enthält, und mit einer unteren Ebene 6, die den Hydraulikteil aufweist.

Die Dialysemaschine 1 ist über eine Zuleitung 7 mit dem Dialysator 8 verbunden, von dem eine Rückleitung 9 zur Dialysemaschine 1 zurückführt, um einen Dialysierflüssigkeitskreislauf zu schließen.

Zum Zwecke einer Dialysebehandlung werden eine arterielle Blutschlauchleitung 10 und eine venöse Blutschlauchleitung 11 an den dargestellten Stellen an dem Dialysator 8 konnektiert. Ein Blutpumpensegment 12 der arteriellen Blutschlauchleitung 10 wird in die Blutpumpe 4 eingelegt, während die venöse Blutschlauchleitung 11 über den Luftdetektor 5 führt.

Zur Vorbereitung einer Dialysebehandlung müssen die Blutschlauchleitungen 10 und 11 freigespült werden und der extrakorporale Blutkreislauf muß entlüftet und befüllt werden. Hierzu ist es bisher üblich, das freie Ende der arteriellen Blutschlauchleitung 10 mit dem Ausgang eines eine Kochsalzlösung enthaltenden Beutels 13 zu verbinden. Die Blutpumpe 4 fördert die Kochsalzlösung durch die arterielle Blutschlauchleitung 10, den Dialysator 8, die venöse Blutschlauchleitung 11 und den Luftdetektor 5, um schließlich die Spülflüssigkeit in einen offenen Behälter 14 abzuführen.

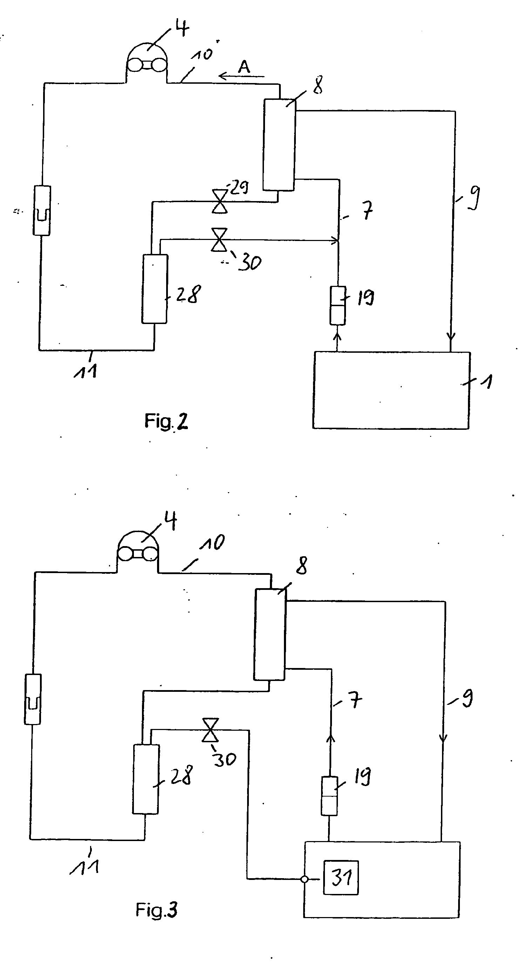

Es wird nun auf die Fig. 2 bis 4 Bezug genommen, in denen die Bauteile, die mit denjenigen gemäß Fig. 1 übereinstimmen, mit denselben Bezugszeichen gekennzeichnet sind. Auf ihre Wiederholung wird aus Gründen der Übersichtlichkeit verzichtet.

Gemäß in Fig. 2 sind bei dem Füll- und Spülvorgang die patientenseitigen Anschlüsse des areriellen und des venösen Blutschlauches 10, 11 miteinander verbunden. In die venöse Blutleitung 11 zwischen dem Dialysator 8 und der venösen Tropfkammer 28 ist ein Ventil 29 in Form einer Schlauchklemme eingefügt. Vom oberen Teil der venösen Tropfkammer 28 zweigt eine ebenfalls mit einem Ventil 30 ausgestattete Leitung ab, die mit einem Auffanggefäß oder, wie in Fig. 2 dargestellt, mit dem Dialysierflüssigkeitssystem verbunden ist.

Zum Zwecke des Füllens und Spülens des extrakorporalen Blutkreislaufes wird die Blutpumpe 4 entgegen ihrer normalen Förderrichtung (Richtungspfeil A) in Tätigkeit gesetzt (z.B. durch Ändern ihrer Drehrichtung), das Ventil 29 wird geschlossen und das Ventil 30 geöffnet.

Durch die Tätigkeit der Blutpumpe entsteht im Blutkompartiment des Dialysators 8 ein Unterdruck. Dadurch tritt filtrierte Dialysierflüssigkeit in das Blutkompartiment und das Blutschlauchsystem über, so daß dieses in kurzer Zeit gefüllt wird. Die abgepumpte Luft und nachfolgende Flüssigkeit werden über das geöffnete Ventil 30 in das Dialysierflüssigkeitssystem gefördert.

Dieses Verfahren kann zur Intensivierung des Spülvorgangs noch dadurch ergänzt werden, daß zwischenzeitlich mehrfach das Ventil 29 geöffnet wird (bei gleichzeitigem oder zeitlich versetztem Schließen des Ventils 30) und bei Bedarf zusätzlich die Blutpumpe in ihrer normalen Förderrichtung (entgegen dem Richtungspfeil A) arbeitet.

Die genannten Umschaltungen der Ventile 29, 30 und der Förderrichtung der Blutpumpe 4 können ohne weiteres über ein automatisch ablaufendes Programm im Rahmen des Steuerungsprogramms des Dialysegerätes bewirkt werden, so daß sich eine erhebliche Arbeitsvereinfachung erreichen läßt.

Fig. 3 zeigt ein weiteres Ausführungsbeispiel. Die patientenseitigen Anschlüsse der Blutschläuche sind zum Zwecke des Füllens und Spülens des extrakorporalen Blutkreislaufes miteinander verbunden. Eine vom oberen Teil der venösen Tropfkammer abzweigende Leitung steht mit einer Saugvorrichtung 31 in Verbindung, die dazu dient, im gesamten extrakorporalen Leitungssystem, einschließlich des Blutkompartiments des Dialysators 8, einen Unterdruck zu erzeugen, so daß filtrierte Dialysierflüssigkeit in das Blutkompartiment übertritt. Bei eingeschalteter Blutpumpe 4 zirkuliert diese Flüssigkeit dann durch den gesamten Kreislauf. Luft und überschüssige Flüssigkeit werden über die vom oberen Teil der venösen Tropfkammer abzweigende Leitung abgeführt.

Die Saugvorrichtung 31 kann eine externe Saugvorrichtung sein, z.B. unter Benutzung des in Krankenhäusern häufig verfügbaren Vakuum-Leitungsnetzes, ggf. mit einem zwischengeschalteten Flüssigkeitsabscheider, oder sie kann als Pumpe ausgebildet sein, wobei die austretende Flüssigkeit in einem Auffanggefäß gesammelt oder in das Dialysierflüssigkeitssystem abgeleitet werden kann. Eine besonders einfache und damit vorteilhafte Anordnung ergibt sich, wenn als Saugvorrichtung das Entgasungssystem des Dialysegerätes mitbenutzt wird. Dabei handelt es sich bei den meisten Dialysegeräten um eine Pumpe, die in einem Teilabschnitt der stromabwärts von ihr liegenden Fließstrecke einen starken Unterdruck erzeugt, um überschüssig gelöstes Gas zur Ausscheidung zu bringen. Die Saugtätigkeit dieser Pumpe kann für den oben beschriebenen Zweck genutzt werden, indem man eine Leitungsverbindung zwischen dem oberen Teil der venösen Tropfkammer und dem genannten Teilabschnitt herstellt.

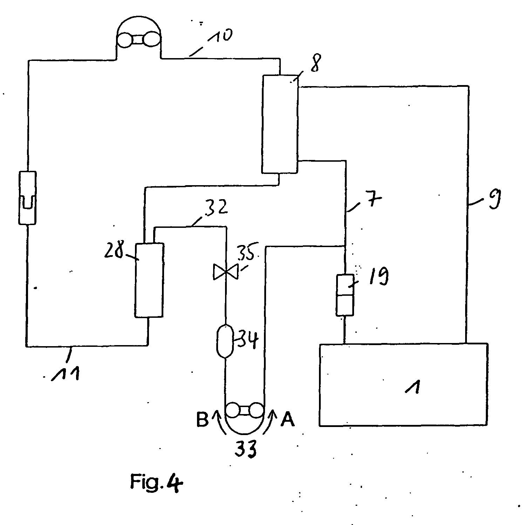

Das Ausführungsbeispiel nach Fig. 4 knüpft an das vorhergehende Beispiel an, bietet aber zusätzliche Funktionen. Der obere Teil der venösen Tropfkammer 28 steht über eine Leitung 32, in die eine Schlauchpumpe 33 eingefügt ist, mit dem Dialysierflüssigkeitssystem in Verbindung. In den Leitungsabschnitt zwischen der venösen Tropfkammer 28 und dieser Pumpe 33 ist außerdem ein Puffergefäß 34 eingefügt, z.B. in Form eines elastischen Beutels mit einem Volumen von typischerweise etwa 20-50 ml. Außerdem ist in den Leitungsabschnitt zwischen der Tropfkammer und dem Puffergefäß 34 ein Ventil 35 eingefügt, vorzugsweise in Form eines Schlauchklemmventils.

Zum Füllen und Spülen des extrakorporalen Blutkreislaufs sind die patientenseitigen Anschlüsse des arteriellen und des venösen Blutschlauches miteinander verbunden. Das Ventil 35 ist geöffnet. Die Pumpe 33 fördert in Richtung A. Dadurch entsteht wie bei dem vorhergehenden Beispiel im Blutschlauchsystem ein Unterdruck, so daß filtrierte Dialysierflüssigkeit durch die Membran des Dialysators 8 in das Blutkompartiment übertritt. Die abgeförderte Luft und die überschüssige Flüssigkeit gelangen über die Pumpe 33 in das Dialysierflüssigkeitssystem.

Nach dem Anschließen des Patienten und dem Beginn der eigentlichen Behandlung dient die in Fig. 4 gezeigte Anordnung dazu, die Dialysemembran entgegen der normalen Filtrationsrichtung periodisch freizuspülen, d.h. Ablagerungen, die sich auf der Blutseite der Membran angesammelt haben, zu entfernen. Es ist bekannt, daß solche Ablagerungen, die eine sogenannte Sekundarmembran bilden und aus Substanzen bestehen, die die Membran nicht passieren können, den effektiven Diffusionswiderstand der Membran vergrößern und dadurch die Effektivität der Blutreinigung, insbesondere hinsichtlich der Ausscheidung höhermoleklarer Schadstoffe, vermindern. Im folgenden wird vorausgesetzt, daß das Dialysierflüssigkeitssystem sich volumenstarr verhält, wie es bei Dialysierflüssigkeitssystemen mit automatischer Bilanzierung (z.B. nach DE-OS 28 38 414) der Fall ist. Das Ventil 35 ist ständig geschlossen. Zum Freispülen der Dialysemembran läuft die Pumpe 33 kurze Zeit (einige Sekunden) mit relativ hoher Fördergeschwindigkeit (in der Größenordnung von 2-10 ml/s) in Richtung A und transportiert Flüssigkeit aus dem Puffergefäß 34 in den Dialysierflüssigkeitskreislauf. Aufgrund des volumenstarren Verhaltens des Dialysierflüssigkeitssystems steigt der Druck im Dialysatkompartiment des Dialysators über den Druck im Blutkompartiment an, so daß eine entsprechende Flüssigkeitsmenge entgegen der normalen Filtrationsrichtung in das Blut übertritt und die aufgelagerten Schichten abschwemmt. In der darauffolgenden Betriebsphase mit normaler Filtrationsrichtung fördert die Pumpe 33 langsam in Richtung B und füllt damit das Puffergefäß 34 wieder auf. Nach einer gewissen Zeit wird die Pumpe wieder in Richtung A in Tätigkeit gesetzt, um das nächste Freispülen der Dialysatormembran einzuleiten. Die Häufigkeit des Freispülens der Dialysatormembran kann nach Bedarf verändert werden. Die Gesamtfiltratbilanz wird von den beschriebenen Vorgängen übrigens nicht beeinflußt, sondern wird, entsprechend der Funktionsweise eines bilanzierenden Dialysierflüssigkeitssystems, von der eingestellten Ultrafiltrationsrate bestimmt.

Die in Fig. 4 gezeigte Anordnung kann darüberhinaus dazu genutzt werden, die Hämodialyse zu einer Hämodiafiltration zu erweitern, d.h. eine Hämodialyse mit wesentlich erhöhter Ultrafiltrationsrate durchzuführen bei gleichzeitiger Substitution des größten Teils der über den Dialysator entzogenen Flüssigkeitsmenge durch Zufuhr einer entsprechenden Menge einer physiologisch angepaßten Lösung in den Blutkreislauf. Bei entsprechender Wahl der Zusammensetzung und gewährleisteter Sterilität der Dialysierflüssigkeit kann diese selbst als Substitutionsflüssigkeit dienen. Zu dem genannten Zweck wird das Ventil 35 geöffnet und die Pumpe 33 mit der Förderrichtung B betrieben. Die Fördergeschwindigkeit wird typischerweise auf 1/3 - 1/5 der Fördergeschwindigkeit der Blutpumpe 4 eingestellt. Unter der Voraussetzung, daß sich das Dialysierflüssigkeitssystem volumenstarr verhält (automatisch bilanzierendes System) entsteht durch die Tätigkeit der Pumpe 33 im Dialysatkompartiment des Dialysators ein zusätzlicher Unterdruck, der eine der Fördergeschwindigkeit der Pumpe 33 entsprechende Filtratmenge aus dem Blutkompartiment in das Dialysatkompartiment übergehen läßt. Die Gesamt-Filtratbilanz wird auch in diesem Falle von den beschriebenen Vorgängen nicht beeinflußt, sondern wird, entsprechend der Funktionsweise eines bilanzierenden Dialysierflüssigkeitssystems, von der eingestellten Ultrafiltrationsrate bestimmt.

Die Anordnung gemäß Fig. 4 kann auch zum Desinfizieren und Spülen des Dialysators verwendet werden. Ein solcher Reinigungszyklus hat zur Folge, daß der Dialysator zur Wiederverwendung aufbereitet werden kann. Dies ist ein weiterer wesentlicher Gesichtspunkt der vorliegenden Erfindung.

Die beschriebenen Funktionen können miteinander kombiniert werden. Eine besonders sinnvolle Kombination ist die Anwendung des periodischen Freispülens der Dialysatormembran bei der Hämodiafiltration, die nach dem beschriebenen oder einem anderen Verfahren durchgeführt wird, weil gerade bei den hohen Filtrationsgeschwindigkeiten, die bei der Hämodiafiltration zur Anwendung gelangen, die Bildung einer den Stoffaustausch hemmenden Sekundärmembran besonders ausgeprägt ist.

Auch andere Teilaspekte der beschriebenen Beispiele können, für den Fachmann leicht ersichtlich, sinnvoll kombiniert werden und durch andere, an sich bekannte Maßnahmen ergänzt werden.

## Patentansprüche

1. Anordnung zum On-Line Freispülen eines extrakorporalen Blutkreislaufs von Dialysemaschinen, insbesondere von Blutschlauchleitungen und Dialysatoren,
**dadurch gekennzeichnet,**
**daß** mit einem automatisch ablaufenden Programm im Rahmen des Steuerprogramms der Dialysemaschine Dialysierflüssigkeit durch die Membran des Dialysators hindurch mit der maschineneigenen Blutpumpe sowie Mitteln zur Erzeugung eines erhöhten Drucks in der Dialysatkammer gegenüber der Blutkammer des Dialysators in den extrakorporalen Blutkreislauf förderbar ist.

## Claims

1. Apparatus for the on-line flushing of an extracorporeal blood circuit of dialysis machines, particularly of blood hoses and dialysers, **characterised in that** dialysis liquid is conveyable into the extracorporeal blood circuit with an automatically running program in the context of the control program of the dialysis machine through the membrane of the dialyser with the blood pump specific to the machine and means for producing an increased pressure in the dialysate chamber with respect to the blood chamber of the dialyser.

## Revendications

1. Ensemble de vidange en ligne d'un circuit de circulation sanguine extracorporelle de machines de dialyse, en particulier des tuyaux flexibles de sang et des dialyseurs,
**caractérisé en ce que**, au moyen d'un programme se déroulant automatiquement dans le cadre du programme de commande de la machine de dialyse, un liquide de dialyse peut être transporté à travers la membrane du dialyseur au moyen de la pompe à sang propre à la machine, ainsi que des moyens destinés à générer une pression plus élevée dans la chambre de dialysat par rapport à la chambre de sang du dialyseur dans le circuit de circulation sanguine extracorporelle.
